Europäisches Patentamt

**European Patent Office**

**Office européen des brevets**

(19)

(11) Publication number: **0 001 904**
A1

# EUROPEAN PATENT APPLICATION

(12)

(21) Application number: **78300550.7**

(22) Date of filing: **27.10.78**

(51) Int. Cl.²: **C 07 C 69/16**
C 07 C 69/28, C 07 C 67/26

(30) Priority: **29.10.77 GB 45128/77**

(43) Date of publication of application:
**16.05.79 Bulletin 79/10**

(84) Designated contracting states:
**BE DE FR GB NL**

(71) Applicant: **BP Chemicals Limited**
**Britannic House Moor Lane**
**London, EC2Y 9BU(GB)**

(72) Inventor: **Randall, George Colin William**
**84 Mill Lane**
**Carshalton Surrey, SM25 2JT(GB)**

(74) Representative: **Harry, John et al,**
**BP TRADING LIMITED Patents & Licensing Division**
**Chertsey Road**
**Sunbury-on-Thames Middlesex TW16 7LN(GB)**

(54) Method of producing alkylene glycol mono acetates and mono propionates.

(57) The present invention relates to a process for producing alkylene glycol monesters from olefin oxides and carboxylic acid using a chromium carboxylate catalyst, chromium octanoate being most preferred. The esters produced using this catalyst are substantially pure and is obained in high yield. The esters thus produced are ideal for use as ester hardeners for foundry cores.

EP 0 001 904 A1

1

## METHOD OF PRODUCING ALKYLENE GLYCOL MONO ACETATES AND MONO PROPIONATES

The present invention relates to a method of producing alkylene monoacetates and monopropionates of high purity and in good yields. Such esters are suitable for use as organic ester hardeners, especially for cold silicates used for bonding sands in the production of foundry cores.

Methods of preparing mono esters of ethyl glycol and propylene glycol particularly of the acrylates and methacrylates have been disclosed in several prior publications, including British patent specification Serial Nos 1133522, 1120301, 1107234, 1082883, and 1054614. The problem with most of these prior art methods is that the final product is a mixture of mono- and diesters, apart from the free acids and alcohols, which are very difficult to separate without resorting to complex fractional distillation techniques. Such expedients not only reduce the overall yield of the monoester recovered but also adversely affect the purity of the product and the economics of the process.

It has now been found that the desired monoesters may be produced in a substantially pure state in relatively high yields without using a complex purification step.

Accordingly, the present invention is a process for producing alkylene glycol monoesters by reacting in the liquid phase a molar excess of ethylene oxide or propylene oxide with acetic acid or propionic acid at elevated temperature in the presence of a catalyst comprising a chromium salt of a saturated or an unsaturated aliphatic carboxylic acid containing between 1 and 10 carbon atoms.

1

The carboxylic acid used in preparing the chromium salt catalyst is suitably selected from formic, acetic, butyric, pentanoic, hexanoic, octanoic, 2-ethylhexanoic and decanoic acids and mixtures thereof. Whichever salt is used, it should preferably be soluble in the reaction medium. Chromium octanoate is a particularly preferred example of such a catalyst.

The chromium salt may be prepared in situ, by heating chromium hydroxide with the carboxylic acid for a short time in order to effect solution. The reaction is then continued in the usual manner. The amount of chromium salt employed in the reaction may vary between 0.1 and 5% by weight of the reactant acid employed.

A slight molar excess of the olefin oxide is used in the reaction mixture. Thus, for example the molar ratio of olefin oxide to the reactant carboxylic acid in the reaction mixture is preferably between 1.05 and 1.2.

The reaction of the present invention may be carried out at a temperature between $40^{\circ}$ and $120^{\circ}$C. The reaction pressure is preferably above atmospheric, for example between 20-40 psig. The process of the present invention may be operated batch-wise or continuously. The course of the reaction may be followed by measuring the acidity of the reaction mixture from time to time and hence determining the content of unreacted acid.

The monoacetates and monopropionates of ethylene glycol and propylene glycol need to have low levels of residual acidity eg less than 0.1% of acetic acid for foundry uses. Such low levels of acidity are obtained at the reaction stage using the process of the present invention whilst maintaining the alkylene glycol monoester content at around 90%. The product may be separated from the catalyst by a simple distillation which may be carried out at reduced pressure.

The invention is further illustrated with reference to the following Examples:

Example 1

Preparation of Propylene Glycol Monoacetate

To a suitable stirred, heated autoclave were charged 360 pt by weight of acetic acid and 5.4 pt by weight of chromium octanoate. The vessel was sealed and evacuated and purged with nitrogen several times to ensure removal of air. The vacuum was finally broken with nitrogen to atmospheric pressure. The vessel contents were heated to 90°C and at this temperature the addition of 418 pt by weight (20% excess) of propylene oxide was commenced at a rate of approximately 15 pt by weight per minute. The temperature was maintained at 90-100°C and the maximum pressure recorded was 28 psig. After 1 hour's reaction the acidity was less than 0.5%, the reactor and contents were cooled to 50°C and vacuum applied for 30 min to remove excess propylene oxide. The crude product was distilled to give a 91% yield of a product containing 93% propylene glycol monoacetate and an acidity of 0.02% as acetic acid.

Example 2

Preparation of Propylene Glycol Monopropionate

To a suitable stirred, heated autoclave were charged 444 pt by weight of propionic acid and 4.5 pt by weight of chromium octanoate. The vessel was sealed and evacuated and purged with nitrogen several times to ensure removal of air. The vacuum was finally broken with nitrogen to atmospheric pressure. The vessel contents were heated to 90°C and at this temperature the addition of 383 pt by weight (10% excess) or propylene oxide was commenced at approximately 15 pt by weight per minute. The temperature was maintained at 90-100°C and the maximum pressure recorded was 34 psig. After 1.7 hr reaction the acidity was less than 0.2%, the reactor and contents were cooled to 50°C and vacuum applied for 30 minutes to remove excess propylene oxide. The crude product was distilled to give a 90% yield of a product containing 97% propylene glycol monopropionate with an acidity of 0.04% as propionic acid.

1. A process for producing alkylene glycol monoesters by reacting in the liquid phase a molar excess of ethylene oxide or propylene oxide with acetic acid or propionic acid at elevated temperature in the presence of a catalyst comprising a chromium salt of a saturated or unsaturated aliphatic carboxylic acid containing between 1 and 10 carbon atoms.

2. A process according to claim 1 wherein the carboxylic acid used in preparing the chromium salt catalyst is selected from formic, acetic, butyric, pentanoic, hexanoic, octanoic, 2-ethylhexanoic and decanoic acids and mixtures thereof.

3. A process according to claim 1 wherein the catalyst is chromium octanoate.

4. A process according to any of the preceding claims 1 to 3 wherein the chromium salt is prepared in situ by heating chromium hydroxide with the carboxylic acid.

5. A process according to any of the preceding claims wherein the amount of chromium salt employed as catalyst in the reaction is between 0.1 and 5% by weight of the reactant acid employed.

6. A process according to any of the preceding claims wherein the molar ratio of alkylene oxide to the reactant carboxylic acid is between 1.05 and 1.2.

7. A process according to any of the preceding claims wherein the reaction is carried out at a temperature between 40 and 120°C.

8. A process according to any of thepreceding claims wherein the reaction is carried out at a pressure of between 20 and 40 psig.

9. A process for producing alkylene glycol monoesters as hereinbefore described with reference to the Examples.

0001904

European Patent
Office

**EUROPEAN SEARCH REPORT**

Application number

EP 78 30 0550

## DOCUMENTS CONSIDERED TO BE RELEVANT

| Category | Citation of document with indication, where appropriate, of relevant passages | Relevant to claim |
|---|---|---|
| | <u>DE - A - 1 911 447</u> (B.P. CHEMICALS) | 1,4-9 |
| | * Page 3; page 4, paragraphs 3,4, 6; page 5, paragraph 1; page 9; claims 1,5; page 10; claims 8,11,12 * | |
| | -- | |
| | <u>US - A - 3 873 602</u> (A. KATZAKIAN et al.) | 1,2,3, 5,7,9 |
| | * Column 4, lines 1-26, lines 57-60; column 5, lines 25-50; column 6, lines 6-10; column 8; claims 4,13; column 9, claim 20 * | |
| | ---- | |

**CLASSIFICATION OF THE APPLICATION (Int. Cl.)**

C 07 C 69/16
     69/28
     67/26

**TECHNICAL FIELDS SEARCHED (Int.Cl.)**

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | 21-12-1978 | KINZINGER |

EPO Form 1503.1 06.78